# EUROPEAN PATENT APPLICATION

(11) **EP 4 800 001 A1**
(43) Date of publication of application: **02.09.2026**
(21) Application number: 24881738.9
(22) Date of filing: 25.10.2024
(51) Int. Cl.: C07C 69/157, C07C 69/78, A61K 31/235, A61K 31/155, A61P 7/02, A61P 9/10, C07C 279/14

(54) **CRYSTAL FORM OF L-ARGININE (S)-2-(1-ACETOXY-N-PENTYL)BENZOATE, AND PREPARATION METHOD THEREFOR AND USE THEREOF**

(30) Priority: 26.10.2023 CN 202311403517
(71) Applicant: Jiangsu Kanion Pharmaceutical Co., Ltd., Lianyungang, Jiangsu 222047 (CN)
(72) Inventor: XIAO, Wei, Lianyungang, Jiangsu 222047 (CN); CHANG, Xinglong, Lianyungang, Jiangsu 222047 (CN); ZHANG, Wei, Lianyungang, Jiangsu 222047 (CN); LI, Yongpeng, Lianyungang, Jiangsu 222047 (CN); HE, Xiaolian, Lianyungang, Jiangsu 222047 (CN); WANG, Zhenzhong, Lianyungang, Jiangsu 222047 (CN); ZHANG, Chenfeng, Lianyungang, Jiangsu 222047 (CN); WANG, Tuanjie, Lianyungang, Jiangsu 222047 (CN)
(74) Representative: Santarelli
(86) International application number: PCT/CN2024/127322
(87) International publication number: WO 2025/087378

(57) **Abstract**

The present invention relates to the technical field of pharmaceuticals. Specifically provided in the present invention are a crystal form of L-arginine (S)-2-(1-acetoxy-n-pentyl)benzoate, and a preparation method therefor and the use thereof. For the crystal form, an X-ray powder diffraction pattern expressed in terms of diffraction angle 2θ is obtained by means of Cu-Kα radiation. The X-ray powder diffraction pattern has characteristic peaks at diffraction angle 2θ of 5.10, 10.23, 14.13, 17.58, 19.81, 20.63 and 21.73, wherein the error range of 2θ of each characteristic peak is ±0.2. The technical solution of the present invention has the following advantages: studies show that the crystal form I of the compound as shown in formula I prepared in the present invention has good stability and high purity, and a single crystal of the crystal form I is obtained; and the crystal form I of the compound as shown in formula I obtained by the technical solution of the present invention can satisfy the medicinal requirements of production, transportation and storage, has a stable, repeatable and controllable production process, and can be adapted to industrial production.

## Description

This application claims the priority of Chinese Patent Application No. 202311403517.0, filed with the China National Intellectual Property Administration on October 26, 2023, and titled with "CRYSTAL FORM OF L-ARGININE *(*S*)*-2-(1-ACETOXY-N-PENTYL)BENZOATE, AND PREPARATION METHOD THEREFOR AND USE THEREOF", which is hereby incorporated by reference in its entirety.

### FIELD

The present disclosure relates to the field of pharmaceutical technology, and more particularly, to a crystal form of L-arginine *(S)*-2-(1-acetoxy-n-pentyl) benzoate, a production method therefor, and a use thereof.

### BACKGROUND

Ischemic cardiovascular and cerebrovascular diseases mainly include transient ischemic attack, cerebral infarction, cerebral artery steal syndrome, and coronary heart disease. Cerebral ischemic stroke refers to ischemic necrosis or softening of localized brain tissue caused by impaired blood supply to the brain, ischemia, and hypoxia. The incidence of ischemic stroke is higher than that of hemorrhagic stroke, accounting for 60% to 70% of the total number of strokes. Occlusion and stenosis of the internal carotid artery and vertebral artery can cause ischemic cerebral stroke. The disease mostly affects individuals over 40 years of age and is more common in men than in women. Severe cases can lead to death. If emergency treatment is not timely or the measures taken are improper, the condition can deteriorate rapidly, becoming life-threatening.

WO2020107500A1 discloses a new class of compounds represented by formula (I) for treating ischemic cardiovascular and cerebrovascular diseases, which have excellent activity and fewer side effects, and therefore have broad prospects for clinical application.

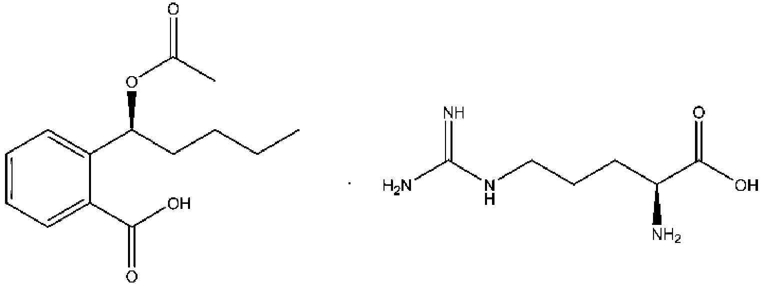

It is well known that a compound can exist in multiple crystal forms. For a compound serving as an active pharmaceutical ingredient, its crystal form often affects the chemical and physical stability of the drug. Differences in the crystal forms, production method, and storage conditions can lead to changes in the crystal structure of the compound, sometimes accompanied by the formation of other crystal forms. Different crystal forms have slightly different physicochemical properties, which can in turn affect the efficacy and stability of the final drug product. Therefore, it is necessary to conduct in-depth research to find a new crystal form that has high crystalline purity and possesses good physicochemical stability, in order to improve various properties of the compound.

### SUMMARY

In view of the foregoing, a technical problem to be solved by the present disclosure is to provide a crystal form of L-arginine *(S)*-2-(1-acetoxy-n-pentyl)benzoate, a production method therefor, and a use thereof, wherein the crystal form has good crystalline stability and chemical stability.

In one aspect, the present disclosure provides a crystal form I of L-arginine *(S)*-2-(1-acetoxy-n-pentyl) benzoate as represented by formula I, wherein an X-ray powder diffraction pattern measured using Cu-Kα radiation has characteristic peaks at diffraction angles 2θ of 5.10, 10.23, 14.13, 17.58, 19.81, 20.63, and 21.73, with an error range of ±0.2 for each characteristic peak 2θ.

In a preferred embodiment, the present disclosure provides a crystal form I of L-arginine *(S)*-2-(1-acetoxy-n-pentyl)benzoate represented by formula I, wherein an X-ray powder diffraction pattern measured using Cu-Kα radiation has characteristic peaks at diffraction angles 2θ of 5.10, 10.23, 14.13, 15.42, 17.58, 19.81, 20.63, 21.73, and 25.88, with an error range of ±0.2 for each characteristic peak 2θ.

In a preferred embodiment, the present disclosure provides a crystal form I of L-arginine *(S)*-2-(1-acetoxy-n-pentyl)benzoate represented by formula I, wherein an X-ray powder diffraction pattern measured using Cu-Kα radiation has characteristic peaks at diffraction angles 2θ of 5.10, 10.23, 14.13, 15.42, 17.58, 19.81, 20.63, 21.73, 24.28, 25.36, 25.88, 31.21, and 31.65, with an error range of ±0.2 for each characteristic peak 2θ.

In a preferred embodiment, the present disclosure provides a crystal form I of L-arginine *(S)*-2-(1-acetoxy-n-pentyl)benzoate represented by formula I, wherein an X-ray powder diffraction pattern measured using Cu-Kα radiation has characteristic peaks at diffraction angles 2θ of 5.10, 10.23, 11.30, 14.13, 15.42, 17.58, 19.81, 20.63, 21.73, 24.28, 25.36, 25.88, 31.21, and 31.65, with an error range of ±0.2 for each characteristic peak 2θ.

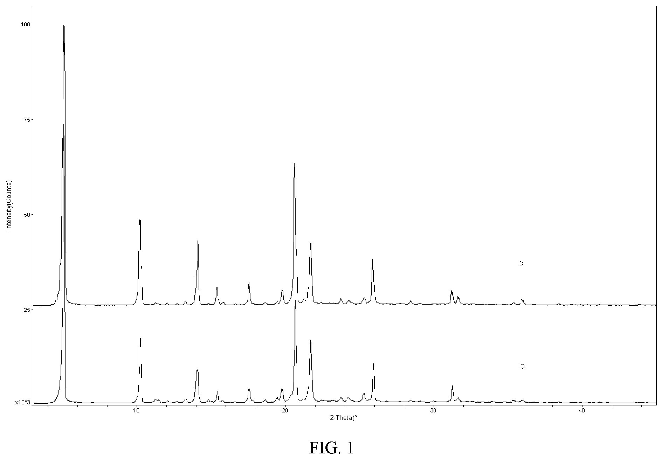

In a preferred embodiment, the present disclosure provides a crystal form I of L-arginine *(S)*-2-(1-acetoxy-n-pentyl)benzoate represented by formula I, wherein an X-ray powder diffraction pattern measured using Cu-Kα radiation is as shown in FIG. 1.

In a preferred embodiment, the present disclosure provides a crystal form I of L-arginine *(S)*-2-(1-acetoxy-n-pentyl)benzoate represented by formula I, wherein an X-ray powder diffraction pattern is measured using Cu-Kα radiation, and parameters of the diffraction angles 2θ are as shown in Table 2.

The present disclosure names the above newly produced crystal form of L-arginine *(S)*-2-(1-acetoxy-n-pentyl)benzoate as crystal form I.

The present disclosure further provides a method for producing the crystal form I of L-arginine *(S)*-2-(1-acetoxy-n-pentyl)benzoate as represented by formula I, wherein the method is selected from any one of Method 1 to Method 7 below:
Method 1: a room temperature evaporation method, comprising dissolving the compound of formula I in a good solvent, allowing the solvent to evaporate from an open container at room temperature, crystallizing, filtering, and drying to obtain the crystal form I of the compound of formula I.

Preferably, in Method 1, the good solvent is a single solvent or a mixed solvent of one or more selected from the group consisting of water, a first alcohol solvent, a sulfoxide solvent, an amide solvent, and a carboxylic acid solvent; wherein the first alcohol solvent is selected from the group consisting of methanol, ethanol, and the like; the sulfoxide solvent is selected from dimethyl sulfoxide; the amide solvent is selected from the group consisting of N,N-dimethylformamide and N,N-dimethylacetamide; and the carboxylic acid solvent is selected from the group consisting of formic acid, acetic acid, and benzoic acid.

More preferably, the good solvent in Method 1 is methanol, dimethyl sulfoxide, or water.

The crystallizing is performed by room temperature crystallization, cooling crystallization, solvent evaporation crystallization, or seed-induced crystallization
Method 2: a suspending crystallization method, comprising mixing the compound of formula I with a poor solvent to form a suspension, shaking the suspension at room temperature or at 40°C to 60°C, removing the solvent, and drying to obtain the crystal form I of the compound of formula I.

Preferably, in Method 2, the poor solvent is a single solvent or a mixed solvent of one or more selected from the group consisting of an ester solvent, an ether solvent, an aliphatic hydrocarbon solvent, an alicyclic hydrocarbon solvent, a second alcohol solvent, a nitrile solvent, and a ketone solvent; wherein the ester solvent is selected from the group consisting of ethyl acetate, isopropyl acetate, and butyl acetate; the ether solvent is selected from the group consisting of tetrahydrofuran, 1,4-dioxane, propylene glycol methyl ether, and methyl tert-butyl ether; the aliphatic hydrocarbon solvent is selected from the group consisting of n-hexane and n-heptane; the alicyclic hydrocarbon solvent is selected from cyclohexane; the second alcohol solvent is selected from the group consisting of isopropanol and the like; the nitrile solvent is selected from acetonitrile; and the ketone solvent is selected from the group consisting of acetone and N-methylpyrrolidone.

More preferably, the poor solvent in Method 2 is isopropanol, acetonitrile, acetone, ethyl acetate, methyl tert-butyl ether, tetrahydrofuran, N-methylpyrrolidone, n-hexane, or cyclohexane.

Method 3: a dissolution-precipitation crystallization method, comprising dissolving the compound of formula I in a good solvent, adding a poor solvent, crystallizing, filtering, and drying to obtain the crystal form I of the compound of formula I.

Preferably, in Method 3, the good solvent is selected from the group consisting of water, a first alcohol solvent, a carboxylic acid solvent, a sulfoxide solvent, and an amide solvent; wherein the first alcohol solvent is selected from the group consisting of methanol, ethanol, and the like; the carboxylic acid solvent is selected from the group consisting of formic acid and acetic acid; the sulfoxide solvent is selected from dimethyl sulfoxide; and the amide solvent is selected from the group consisting of N,N-dimethylformamide and N,N-dimethylacetamide; the poor solvent is selected from the group consisting of a second alcohol solvent, an ether solvent, an ester solvent, a ketone solvent, and a nitrile solvent; wherein the second alcohol solvent is selected from the group consisting of isopropanol and the like; the ether solvent is selected from the group consisting of tetrahydrofuran, 1,4-dioxane, propylene glycol methyl ether, and methyl tert-butyl ether; the ester solvent is selected from the group consisting of ethyl acetate, isopropyl acetate, and butyl acetate; the ketone solvent is selected from the group consisting of acetone and N-methylpyrrolidone; and the nitrile solvent is selected from acetonitrile.

More preferably, the good solvent is selected from the group consisting of methanol, ethanol, acetic acid, dimethyl sulfoxide, N,N-dimethylformamide, and water.

More preferably, the poor solvent is selected from the group consisting of isopropanol, acetonitrile, acetone, ethyl acetate, isopropyl acetate, methyl tert-butyl ether, tetrahydrofuran, and N-methylpyrrolidone.

In some specific embodiments of the present disclosure, the good solvent-poor solvent combination may be specifically: methanol-isopropanol, methanol-acetonitrile, methanol-acetone, methanol-ethyl acetate, methanol-methyl tert-butyl ether, methanol-tetrahydrofuran, ethanol-isopropanol, ethanol-acetonitrile, ethanol-acetone, ethanol-ethyl acetate, ethanol-methyl tert-butyl ether, ethanol-tetrahydrofuran, acetic acid-isopropanol, acetic acid-acetonitrile, acetic acid-ethyl acetate, acetic acid-isopropyl acetate, acetic acid-tetrahydrofuran, water-isopropanol, water-acetonitrile, water-acetone, or water-tetrahydrofuran.

Preferably, the crystallizing is performed by a method selected from the group consisting of room temperature crystallization, cooling crystallization, solvent evaporation crystallization, and seed-induced crystallization.

Method 4: a binary mixed-solvent crystallization method, comprising dissolving the compound of formula I in a binary mixed solvent, crystallizing, filtering, and drying to obtain the crystal form I of the compound of formula I, wherein the binary mixed solvent is a mixed solvent of a good solvent and a poor solvent.

Preferably, in Method 4, the good solvent is selected from the group consisting of a first alcohol solvent and water; wherein the first alcohol solvent is selected from the group consisting of methanol and ethanol; the poor solvent is selected from the group consisting of a second alcohol solvent, a nitrile solvent, a ketone solvent, an ester solvent, and an ether solvent; wherein the second alcohol solvent is selected from isopropanol; the nitrile solvent is selected from acetonitrile; the ketone solvent is selected from the group consisting of acetone and N-methylpyrrolidone; the ester solvent is selected from the group consisting of ethyl acetate, isopropyl acetate, and butyl acetate; and the ether solvent is selected from the group consisting of tetrahydrofuran, 1,4-dioxane, propylene glycol methyl ether, and methyl tert-butyl ether.

More preferably, the binary mixed solvent is a mixed solvent of methanol and a poor solvent, wherein the poor solvent is preferably selected from the group consisting of isopropanol, acetonitrile, acetone, ethyl acetate, isopropyl acetate, methyl tert-butyl ether, tetrahydrofuran, and N-methylpyrrolidone.

More preferably, the binary mixed solvent is a mixed solvent of water and a poor solvent, wherein the poor solvent is preferably selected from the group consisting of isopropanol, acetonitrile, acetone, methyl tert-butyl ether, tetrahydrofuran, and N-methylpyrrolidone.

In some specific embodiments of the present disclosure, the binary mixed solvent may be a mixed solvent of methanol-isopropanol, ethanol-acetonitrile, methanol-acetone, methanol-ethyl acetate, methanol-methyl tert-butyl ether, methanol-tetrahydrofuran, methanol-N-methylpyrrolidone, water-isopropanol, water-acetonitrile, water-acetone, water-methyl tert-butyl ether, water-tetrahydrofuran, or water-N-methylpyrrolidone.

A volume ratio of the good solvent to the poor solvent is preferably 1:5 to 5:1; in some specific embodiments, the volume ratio of the good solvent to the poor solvent is 1:4, 1:1, or 4:1.

Method 5: a grinding crystallization method, comprising mixing and grinding the compound of formula I with a poor solvent to obtain the crystal form I of the compound of formula I.

Preferably, in Method 5, the poor solvent is selected from the group consisting of a second alcohol solvent, a nitrile solvent, a ketone solvent, an ester solvent, an ether solvent, a ketone solvent, an aliphatic hydrocarbon solvent, and an alicyclic hydrocarbon solvent; wherein the second alcohol solvent is selected from the group consisting of isopropanol and the like; the nitrile solvent is selected from acetonitrile; the ketone solvent is selected from the group consisting of acetone and N-methylpyrrolidone; the ester solvent is selected from the group consisting of ethyl acetate, isopropyl acetate, and butyl acetate; the ether solvent is selected from the group consisting of tetrahydrofuran, 1,4-dioxane, propylene glycol methyl ether, and methyl tert-butyl ether; the aliphatic hydrocarbon solvent is selected from the group consisting of n-hexane and n-heptane; and the alicyclic hydrocarbon solvent is selected from cyclohexane.

More preferably, the said poor solvent is selected from the group consisting of isopropanol, acetonitrile, acetone, ethyl acetate, isopropyl acetate, methyl tert-butyl ether, tetrahydrofuran, N-methylpyrrolidone, n-hexane, and cyclohexane.

In the present disclosure, the compound of formula I is mixed and ground with a poor solvent; after 30 minutes, crystal form formation is determined by XRPD pattern measurement.

Method 6: a rapid cooling crystallization method, comprising dissolving the compound of formula I in a good solvent, and crystallizing at a low temperature, filtering, and drying to obtain the crystal form I of the compound of formula I;

Preferably, in Method 6, the said good solvent is selected from the group consisting of a first alcohol solvent, a carboxylic acid solvent, a sulfoxide solvent, an amide solvent, and water; wherein the first alcohol solvent is selected from the group consisting of methanol, ethanol, and the like; the sulfoxide solvent is selected from dimethyl sulfoxide; the amide solvent is selected from the group consisting of N,N-dimethylformamide and N,N-dimethylacetamide; and the carboxylic acid solvent is selected from the group consisting of formic acid, acetic acid, and benzoic acid.

More preferably, the good solvent is selected from methanol.

Preferably, the low temperature is provided by an ice-water mixture.

Method 7: a vapor diffusion method, comprising dissolving the compound of formula I in a good solvent, placing the solution in an open container in an atmosphere of a poor solvent, allowing the solution to stand at room temperature, crystallizing, filtering, and drying to obtain the crystal form I of the compound of formula I.

Preferably, in Method 7, the good solvent is selected from the group consisting of water, a first alcohol solvent, and a carboxylic acid solvent; wherein the first alcohol solvent is selected from the group consisting of methanol and ethanol; and the carboxylic acid solvent is selected from the group consisting of formic acid and acetic acid; the poor solvent is selected from the group consisting of a second alcohol solvent, an ether solvent, an ester solvent, a ketone solvent, and a nitrile solvent; wherein the second alcohol solvent is selected from isopropanol; the ether solvent is selected from the group consisting of tetrahydrofuran, 1,4-dioxane, propylene glycol methyl ether, and methyl tert-butyl ether; the ester solvent is selected from the group consisting of ethyl acetate, isopropyl acetate, and butyl acetate; the ketone solvent is selected from the group consisting of acetone and N-methylpyrrolidone; and the nitrile solvent is selected from acetonitrile.

More preferably, the said good solvent is selected from the group consisting of methanol, formic acid, acetic acid, and water.

More preferably, the said poor solvent is selected from the group consisting of isopropanol, acetonitrile, acetone, ethyl acetate, isopropyl acetate, methyl tert-butyl ether, and tetrahydrofuran.

In some specific embodiments of the present disclosure, the good solvent-poor solvent combinations may be: methanol-isopropanol, water-isopropanol, methanol-acetonitrile, water-acetonitrile, methanol-acetone, acetic acid-acetone, water-acetone, methanol-ethyl acetate, water-ethyl acetate, methanol-isopropyl acetate, water-isopropyl acetate, methanol-methyl tert-butyl ether, water-methyl tert-butyl ether, or methanol-tetrahydrofuran.

The aforementioned placing in an open container in an atmosphere of a poor solvent specifically comprises: after dissolving the compound of formula I in a good solvent, placing the solution in an open container inside another larger container (outer container), adding a poor solvent to the outer container, and capping and tightening the outer container or sealing it with a sealing film.

The crystallizing can be performed by conventional crystallization methods, such as room temperature crystallization, cooling crystallization, solvent evaporation crystallization, and seed-induced crystallization.

In the present disclosure, the produced crystal form I is subjected to structural determination and crystal form studies by X-ray powder diffraction (XRPD) and differential scanning calorimetry (DSC).

In the present disclosure, the produced L-arginine *(S)*-2-(1-acetoxy-n-pentyl)benzoate crystal form I was subjected to DSC and thermogravimetric analysis (TGA). The DSC trace results show that the L-arginine *(S)*-2-(1-acetoxy-n-pentyl)benzoate crystal form I has a melting point of 192°C.

The TGA results show that the L-arginine *(S)*-2-(1-acetoxy-n-pentyl)benzoate crystal form I has a decomposition temperature of 195°C.

Stability study results show that after the L-arginine *(S)*-2-(1-acetoxy-n-pentyl) benzoate crystal form I produced in the present disclosure was placed for 10 days under conditions of high temperature (60°C), high humidity (RH 90%±5%), and light exposure (4500 lx ± 500 lx, with the total illumination from the light source reaching 1.2×10⁶ lux•hr), its powder diffraction pattern, DSC trace, and TGA trace showed no significant difference, indicating good stability and no crystal form transformation.

The methods for crystallization of the crystal form in the present disclosure are conventional, for example, evaporation crystallization, cooling crystallization, or crystallization at room temperature.

The starting material used in the method for producing crystal form I as described in the present disclosure can be the compound of formula I in any form, including but not limited to: an amorphous form, any crystal form, and the like.

The present disclosure also provides a method for producing a single crystal of L-arginine *(S)*-2-(1-acetoxy-n-pentyl) benzoate crystal form I, comprising:
dissolving L-arginine *(S)*-2-(1-acetoxy-n-pentyl) benzoate in a water-acetone mixed solvent, and allowing slow evaporation of the solvent, to obtain colorless, transparent, strip-like crystals.

A volume ratio of water to acetone is preferably 1:4 to 4:1.

The slow evaporation is performed for preferably 15 to 25 days, more preferably 20 days.

The colorless, transparent, strip-like crystals obtained in the present disclosure meet the requirements for a single-crystal X-ray diffraction experiment.

In another aspect, the present disclosure provides a pharmaceutical composition, comprising the aforementioned crystal form I of L-arginine *(S)*-2-(1-acetoxy-n-pentyl) benzoate represented by formula I and a pharmaceutically acceptable adjuvant.

The aforementioned excipient includes, but is not limited to, a carrier, a diluent, or an excipient, and the like.

The pharmaceutical composition may be made into any pharmaceutically acceptable dosage form, including but not limited to, a tablet, a capsule, a pill, a granule, a solution, a suspension, a syrup, an injection (including a solution for injection, a sterile powder for injection, and a concentrated solution for injection), a suppository, an inhalant, or a spray, and the like.

The pharmaceutical composition of the present disclosure may be administered to a patient or subject in need of such treatment by any appropriate route of administration, for example, oral, parenteral, rectal, pulmonary, or topical administration. When used for oral administration, the pharmaceutical composition may be made into an oral preparation, for example, an oral solid preparation, such as a tablet, a capsule, a pill, a granule, and the like; or an oral liquid preparation, such as an oral solution, an oral suspension, a syrup, and the like. When made into an oral preparation, the pharmaceutical preparation may also contain an appropriate filler, a binder, a disintegrant, a lubricant, and the like. When used for parenteral administration, the pharmaceutical preparation may be made into an injection, including a solution for injection, a sterile powder for injection, and a concentrated solution for injection. When made into an injection, the pharmaceutical composition can be produced by conventional methods in the pharmaceutical field. When formulating an injection, no additional additives may be added to the pharmaceutical preparation, or an appropriate additional additive may be added according to the properties of the drug. When used for rectal administration, the pharmaceutical preparation can be made into a suppository and the like. When used for pulmonary administration, the pharmaceutical preparation can be made into an inhalant or a spray, and the like. In some preferred embodiments, the crystal form I of the compound of formula I of the present disclosure is present in a therapeutically and/or prophylactically effective amount in the pharmaceutical composition or medicament. In some preferred embodiments, the crystal form I of the compound of formula I of the present disclosure is present in a unit dosage form in the pharmaceutical composition or medicament.

In another aspect, the present disclosure provides a use of the aforementioned crystal form I of L-arginine *(S)*-2-(1-acetoxy-n-pentyl)benzoate as represented by formula I or the aforementioned pharmaceutical composition in the manufacture of a medicament for treating an ischemic cardiovascular and cerebrovascular disease.

The ischemic cardiovascular and cerebrovascular disease is preferably stroke; more preferably cerebral stroke; and further preferably ischemic cerebral stroke.

In another aspect, the present disclosure provides a method for treating an ischemic cardiovascular or cerebrovascular disease, comprising administering to a subject in need thereof the crystal form I of the compound of formula I of the present disclosure, or the pharmaceutical composition of the present disclosure, at a therapeutically and/or prophylactically effective amount.

In the specification and claims of the present disclosure, unless otherwise indicated, the scientific and technical terms used herein have the meanings commonly understood by one of ordinary skill in the art. However, for a better understanding of the present disclosure, definitions and explanations of some related terms are provided below. In addition, when the definitions and explanations of terms provided in the present disclosure are inconsistent with the meanings commonly understood by one of ordinary skill in the art, the definitions and explanations of the terms provided in the present disclosure shall prevail.

The "ether solvent" as used in the present disclosure refers to a chain compound or cyclic compound containing an ether bond -O- and having 1 to 10 carbon atoms; specific examples include, but are not limited to: tetrahydrofuran, diethyl ether, propylene glycol methyl ether, methyl tert-butyl ether, or 1,4-dioxane.

The "alcohol solvent" as used in the present disclosure refers to a group derived from the substitution of one or more hydrogen atoms on a "C₁₋₆ alkyl" with one or more "hydroxyl groups," wherein the "hydroxyl group" and "C₁₋₆ alkyl" are as defined previously; specific examples include, but are not limited to: methanol, ethanol, isopropanol, n-propanol, isoamyl alcohol, or trifluoroethanol.

The "ester solvent" as used in the present disclosure refers to a compound formed by a low-grade organic acid having 1 to 4 carbon atoms and a low-grade alcohol having 1 to 6 carbon atoms; specific examples include, but are not limited to: ethyl acetate, isopropyl acetate, or butyl acetate.

The "ketone solvent" as used in the present disclosure refers to a compound in which a carbonyl group (-C(O)-) is connected to two hydrocarbyl groups; depending on the hydrocarbyl groups in the molecule, a ketone can be classified into an aliphatic ketone, an alicyclic ketone, an aromatic ketone, a saturated ketone, and an unsaturated ketone; specific examples include, but are not limited to: acetone, acetophenone, methyl isobutyl ketone, or N-methylpyrrolidone.

The "nitrile solvent" as used in the present disclosure refers to a group derived from the substitution of one or more hydrogen atoms on a "C₁₋₆ alkyl" with one or more "cyano groups," wherein the "cyano group" and "C₁₋₆ alkyl" are as defined previously; specific examples include, but are not limited to: acetonitrile or propionitrile.

The "aliphatic hydrocarbon solvent" as used in the present disclosure refers to a hydrocarbon having the basic properties of an aliphatic compound, in which the carbon atoms are linked to form an open-chain carbon skeleton rather than a ring, and having 1 to 10 carbon atoms, such as saturated aliphatic hydrocarbons, including alkane solvents; specific examples include, but are not limited to: n-butane, n-pentane, n-hexane, or n-heptane.

The "alicyclic hydrocarbon solvent" as used in the present disclosure refers to a hydrocarbon compound having a cyclic carbon skeleton, properties similar to those of aliphatic hydrocarbons, and 1 to 8 ring atoms; specific examples include, but are not limited to: cyclopentane or cyclohexane.

The "amide solvent" as used in the present disclosure refers to a compound containing a carbonylamino group (-C(O)N-) and having 1 to 10 carbon atoms; specific examples include, but are not limited to: N,N-dimethylformamide or N,N-dimethylacetamide.

The "mixed solvent" as used in the present disclosure refers to a solvent formed by mixing one or more different types of organic solvents in a certain ratio, or a solvent formed by mixing an organic solvent and water in a certain ratio; the mixed solvent is preferably a mixed solvent of one or more alcohols, a mixed solvent of an alcohol and an ether, a mixed solvent of an alcohol and an aliphatic hydrocarbon, a mixed solvent of an ether and an aliphatic hydrocarbon, a mixed solvent of an alcohol and water, a mixed solvent of a halogenated hydrocarbon and a nitrile solvent, a mixed solvent of an amide solvent and water, or a mixed solvent of an ether and water; wherein the alcohol, ether, aliphatic hydrocarbon, halogenated hydrocarbon, amide, and nitrile are as defined previously.

The "X-ray powder diffraction pattern or XRPD" as used in the present disclosure refers to a pattern based on the Bragg equation 2d sin θ = nλ (wherein λ is the wavelength of the X-ray; λ=1.54056 Å; and n, the order of diffraction, is any positive integer, and generally the first-order diffraction peak is taken, so n = 1); when X-rays are incident at a glancing angle θ (the complementary angle of the angle of incidence, also known as the Bragg angle) onto a set of atomic planes having a lattice plane spacing d in a crystal or partial crystal sample, the Bragg equation is satisfied, whereby this set of X-ray powder diffraction patterns is measured.

The "differential scanning calorimetry or DSC" as used in the present disclosure refers to a technique for measuring the temperature difference or heat flow difference between a sample and a reference during the heating or isothermal holding process of the sample, to characterize all physical and chemical changes associated with thermal effects, thereby obtaining phase transition information of the sample.

The "2θ or 2θ angle" as used in the present disclosure refers to a diffraction angle, where θ is the Bragg angle, the unit is ° or degrees, and the error range for 2θ is ±0.1 to ±0.5, preferably ±0.1 to ±0.3, and more preferably ±0.2.

The "interplanar spacing or d-spacing (d-value)" as used in the present disclosure refers to three non-parallel unit vectors a, b, and c, selected in a space lattice and connecting two adjacent lattice points, and they divide the lattice into juxtaposed parallelepiped units, which is referred to as interplanar spacing. The space lattice is divided according to the lines connecting the determined parallelepiped units to obtain a set of straight line grids, called a space lattice or crystal lattice. The point lattice and crystal lattice respectively reflect the periodicity of the crystal structure with geometric points and lines; different crystal planes have different interplanar spacings (i.e., the distance between two adjacent parallel crystal planes); the unit is Å or Angstrom.

Compared to the prior art, the present disclosure provides the crystal form I of L-arginine *(S)*-2-(1-acetoxy-n-pentyl)benzoate as represented by formula I, wherein an X-ray powder diffraction pattern measured using Cu-Kα radiation has characteristic peaks at diffraction angles 2θ of 5.10, 10.23, 14.13, 17.58, 19.81, 20.63, and 21.73, with an error range of ±0.2 for each characteristic peak 2θ.

The technical solutions of the present disclosure have the following advantages:
Studies have shown that the crystal form I of the compound of formula I produced in the present disclosure has good stability and high purity, and a single crystal of crystal form I has been obtained, which can be better applied clinically. The crystal form I of the compound of formula I obtained by the technical solutions of the present disclosure can meet the pharmaceutical requirements for production, transportation, and storage; the production process is stable, reproducible, and controllable, and is adaptable to industrial-scale production.

### BRIEF DESCRIPTION OF DRAWINGS

FIG. 1 is a PXRD pattern of crystal form I;
FIG. 2 is a DSC trace of crystal form I;
FIG. 3 is a TGA trace of crystal form I;
FIG. 4 is an ellipsoid plot of the stereochemical structure of an asymmetric unit of a single crystal of the compound represented by formula I;
FIG. 5 is an ellipsoid plot of the stereochemical structure of a single molecule of a single crystal of the compound represented by formula I;
FIG. 6 is a projection diagram of the crystal cell packing of a single crystal of the compound represented by formula I, viewed along the a-axis;
FIG. 7 shows the comparison between a simulated powder diffraction pattern from the single crystal structure of the compound represented by formula I and the experimental PXRD pattern of crystal form I;
FIG. 8 is a PXRD overlay plot of crystal form I (high temperature);
FIG. 9 is a DSC overlay plot of crystal form I (high temperature);
FIG. 10 is a TGA overlay plot of crystal form I (high temperature);
FIG. 11 is a PXRD overlay plot of crystal form I (high humidity);
FIG. 12 is a DSC overlay plot of crystal form I (high humidity);
FIG. 13 is a TGA overlay plot of crystal form I (high humidity);
FIG. 14 is a PXRD overlay plot of crystal form I (light exposure);
FIG. 15 is a DSC overlay plot of crystal form I (light exposure);
FIG. 16 is a TGA overlay plot of crystal form I (light exposure); and
FIG. 17 is a PXRD pattern of the compound represented by formula I prepared in Comparative Example 1.

### DETAILED DESCRIPTION

To further illustrate the present disclosure, the crystal form of L-arginine *(S)*-2-(1-acetoxy-n-pentyl)benzoate, the production method therefor, and the use thereof provided by the present disclosure are described in detail below in conjunction with examples. The examples of the present disclosure are only for illustrating the technical solutions of the present disclosure and are not intended to limit the substance and scope of the present disclosure.

The test conditions for the instruments used in the experiments were:

### 1. Differential Scanning Calorimeter (DSC)

Instrument model: Q2000 DSC 3+Q500 TGA;
Purge gas: Nitrogen;
Heating rate: 10.0°C/min;
Temperature range: 30°C to 300°C;

The temperature range for thermogravimetric analysis (TGA) was 30°C to 350°C, with a heating rate of 10°C/min.

### 2. X-ray Powder Diffraction (XRPD)

Instrument model: D8 Advance X-ray powder diffractometer;
Radiation: Monochromatic Cu-Kα radiation (λ = 1.5406 Å);
Scan mode: θ/2θ, Scan range: 3° to 45°;
Voltage: 40 kV, Current: 40 mA.

### 3. Single-crystal X-ray Diffractometer

Instrument model: SMART APEX-II.

The compound represented by formula I below was prepared according to the method disclosed in Example 10 of WO2020107500A1.

### Example 1 Room Temperature Evaporation Method (Single Solvent)

50 mg of the compound represented by formula I was weighed and placed in a reaction flask. A solvent was added to achieve complete dissolution. The solution was then placed in a fume hood to allow slow evaporatation of the solvent at room temperature. The solid of the compound represented by formula I was obtained after filtration. The solid was analyzed by XRPD, and the results are shown in Table 1:

**Table 1 Experimental Results of Room Temperature Evaporation Method (Single Solvent)**

| **Solvent** | **XRPD Result** |
|---|---|
| Methanol | Crystal form I |
| Acetic acid | Amorphous + Crystal form I |
| Dimethyl sulfoxide | Crystal form I |
| N,N-Dimethylformamide | Amorphous + Crystal form I |
| Water | Crystal form I |
| Formic acid | Amorphous |

The prepared crystal form I was analyzed by XRD, and the results are shown in Table 2:

**Table 2 Characteristic Peaks of Crystal Form I**

| **Peak No.** | **2θ (°)** | **d (Å)** |
|---|---|---|
| 1 | 5.096 | 17.3275 |
| 2 | 10.229 | 8.6405 |
| 3 | 11.293 | 7.8287 |
| 4 | 11.457 | 7.7174 |
| 5 | 12.068 | 7.3279 |
| 6 | 12.752 | 6.936 |
| 7 | 13.313 | 6.6451 |
| 8 | 14.125 | 6.2648 |
| 9 | 14.804 | 5.9791 |
| 10 | 15.423 | 5.7404 |
| 11 | 15.842 | 5.5894 |
| 12 | 17.583 | 5.0397 |
| 13 | 17.835 | 4.9692 |
| 14 | 18.684 | 4.7452 |
| 15 | 19.459 | 4.558 |
| 16 | 19.814 | 4.4771 |
| 17 | 20.627 | 4.3025 |
| 18 | 21.293 | 4.1692 |
| 19 | 21.73 | 4.0865 |
| 20 | 23.77 | 3.7402 |
| 21 | 24.281 | 3.6626 |
| 22 | 25.362 | 3.5088 |
| 23 | 25.883 | 3.4394 |
| 24 | 28.467 | 3.1329 |
| 25 | 29.061 | 3.0702 |
| 26 | 31.214 | 2.8631 |
| 27 | 31.65 | 2.8247 |
| 28 | 35.42 | 2.5321 |
| 29 | 35.941 | 2.4966 |

FIG. 1 shows a PXRD pattern of crystal form I, wherein curves a and b are the PXRD patterns of different batches of crystal form I prepared using methanol as the solvent.

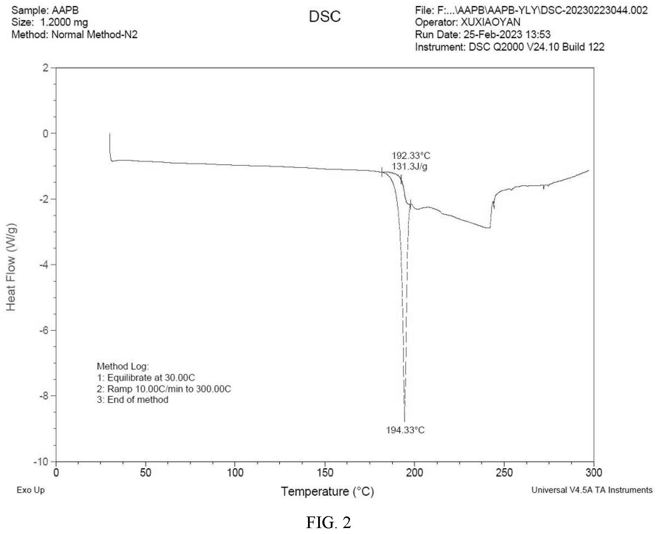

FIG. 2 shows a DSC trace of crystal form I prepared using methanol as the solvent.

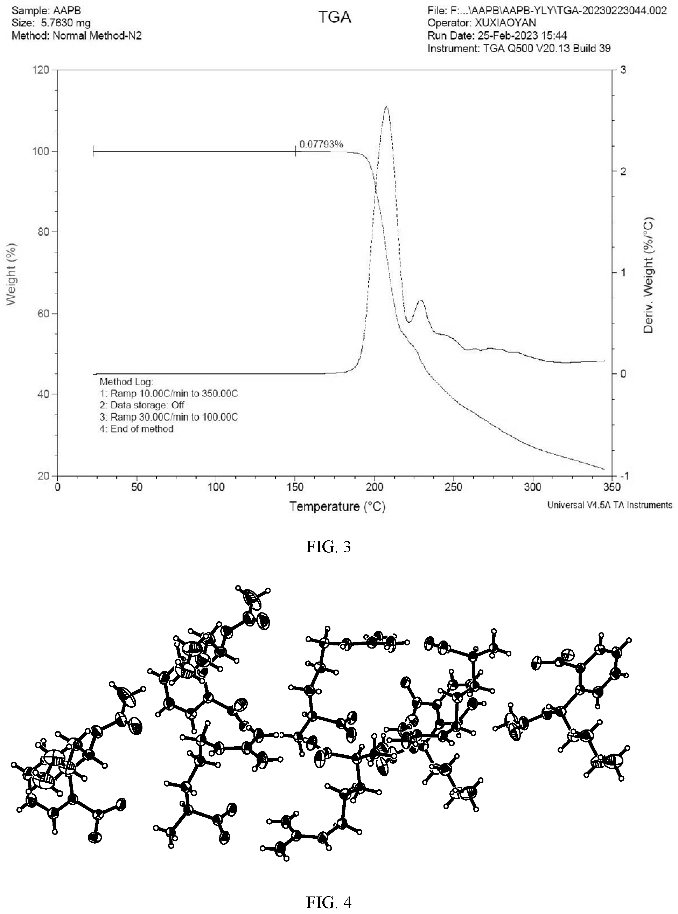

FIG. 3 shows a TGA trace of crystal form I prepared using methanol as the solvent.

The results from FIG. 1 to FIG. 3 indicated that the two batches of samples were both crystalline and of a consistent crystal form, and did not contain any crystalline solvent or crystal water.

### Example 2 Suspending Crystallization Method

30 mg of the compound represented by formula I was weighed and placed in a reaction flask, and a solvent was added to form a suspension. The suspension was then shaken for 48 hours under conditions of room temperature or 50°C, respectively. The solvent was removed, and the solid of the compound represented by formula I was obtained after drying. The solid was analyzed by PXRD, and the results are shown in Table 3:

**Table 3 Experimental Results of Suspending Crystallization Method**

| **Solvent** | **Temperature (°C)** | **PXRD Result** |
|---|---|---|
| Isopropanol | Room temperature | Crystal form I |
| | 50 | Crystal form I |
| Acetonitrile | Room temperature | Crystal form I |
| | 50 | Crystal form I |
| Acetone | Room temperature | Crystal form I |
| | 50 | Crystal form I |
| Ethyl acetate | Room temperature | Crystal form I |
| | 50 | Crystal form I |
| Isopropyl acetate | Room temperature | Mixed crystal forms |
| | 50 | Mixed crystal forms |
| Methyl tert-butyl ether | Room temperature | Crystal form I |
| | 50 | Crystal form I |
| Tetrahydrofuran | Room temperature | Crystal form I |
| | 50 | Crystal form I |
| N-Methylpyrrolidone | Room temperature | Crystal form I |
| | 50 | Crystal form I |
| n-Hexane | Room temperature | Crystal form I |
| | 50 | Crystal form I |
| Cyclohexane | Room temperature | Crystal form I |
| | 50 | Crystal form I |

### Example 3 Dissolution-Precipitation Crystallization Method

50 mg of the compound represented by formula I was weighed into a reaction flask and was dissolved in a relatively good solvent to prepare a solution. A poor solvent was then gradually added to the solution. If a solid precipitated, it was analyzed by PXRD. If no solid precipitated by the time the total solvent volume reached 20 mL, the solvent addition dropwise was stopped, and the solution was left to stand at room temperature for natural evaporation. The solid obtained after evaporation was then analyzed by PXRD. The results are shown in Table 4:

**Table 4 Experimental Results of Dissolution-Precipitation Crystallization Method**

| **Solvent** | **PXRD Result** |
|---|---|
| Methanol-isopropanol | Crystal form I |
| Methanol-acetonitrile | Crystal form I |
| Methanol-acetone | Crystal form I |
| Methanol-ethyl acetate | Crystal form I |
| Methanol-isopropyl acetate | Mixed crystal forms |
| Methanol-methyl tert-butyl ether | Crystal form I |
| Methanol-tetrahydrofuran | Crystal form I |
| Methanol-N-methylpyrrolidone | Amorphous |
| Ethanol-isopropanol | Crystal form I |
| Ethanol-acetonitrile | Crystal form I |
| Ethanol-acetone | Crystal form I |
| Ethanol-ethyl acetate | Crystal form I |
| Ethanol-isopropyl acetate | Mixed crystal forms |
| Ethanol-methyl tert-butyl ether | Crystal form I |
| Ethanol-tetrahydrofuran | Crystal form I |
| Ethanol-N-methylpyrrolidone | Amorphous |
| Acetic acid-isopropanol | Crystal form I |
| Acetic acid-acetonitrile | Crystal form I |
| Acetic acid-acetone | Mixed crystal forms |
| Acetic acid-ethyl acetate | Crystal form I |
| Acetic acid-isopropyl acetate | Crystal form I |
| Acetic acid-methyl tert-butyl ether | Mixed crystal forms |
| Acetic acid-tetrahydrofuran | Crystal form I |
| Acetic acid-N-methylpyrrolidone | Amorphous |
| Dimethyl sulfoxide-isopropanol | Amorphous |
| Dimethyl sulfoxide-acetonitrile | NS |
| Dimethyl sulfoxide-acetone | NS |
| Dimethyl sulfoxide-ethyl acetate | NS |
| Dimethyl sulfoxide-isopropyl acetate | Amorphous |
| Dimethyl sulfoxide-methyl tert-butyl ether | Amorphous |
| Dimethyl sulfoxide-tetrahydrofuran | Amorphous |
| Dimethyl sulfoxide-N-methylpyrrolidone | Amorphous |
| N,N-Dimethylformamide-isopropanol | Amorphous |
| N,N-Dimethylformamide-acetonitrile | Amorphous |
| N,N-Dimethylformamide-acetone | Amorphous |
| N,N-Dimethylformamide-ethyl acetate | Amorphous |
| N,N-Dimethylformamide-isopropyl acetate | Amorphous |
| N,N-Dimethylformamide-methyl tert-butyl ether | Amorphous |
| N,N-Dimethylformamide-tetrahydrofuran | Amorphous |
| N,N-Dimethylformamide-N-methylpyrrolidone | Amorphous |
| Water-isopropanol | Crystal form I |
| Water-acetonitrile | Crystal form I |
| Water-acetone | Crystal form I |
| Water-tetrahydrofuran | Crystal form I |
| Water-N-methylpyrrolidone | Mixed crystal forms |

| | |
|---|---|
| NS indicates that no solid was obtained during the experiment. | |

### Example 4 Binary Mixed-Solvent Crystallization Method

50 mg of the compound represented by formula I was weighed and placed in a reaction flask, and a binary mixed solvent (mixed at volume ratios of 1:4, 1:1, or 4:1) was added. If a clear solution was obtained, it was placed at room temperature to allow slow evaporation, and the resulting solid was analyzed by PXRD. If a suspension was obtained, it was allowed to stand at room temperature, the solvent was then removed, and the resulting solid was analyzed by PXRD. The results are shown in Table 5:

**Table 5 Experimental Results of Binary Mixed-Solvent Crystallization Method**

| **Solvent** | **Ratio (v/v)** | **Result** |
|---|---|---|
| Methanol-isopropanol | 1:1 | Crystal form I |
| Methanol-isopropanol | 1:4 | Crystal form I |
| Methanol-isopropanol | 4:1 | Crystal form I |
| Methanol-acetonitrile | 1:1 | Crystal form I |
| Methanol-acetonitrile | 1:4 | Crystal form I |
| Methanol-acetonitrile | 4:1 | Crystal form I |
| Methanol-acetone | 1:1 | Crystal form I |
| Methanol-acetone | 1:4 | Crystal form I |
| Methanol-acetone | 4:1 | Crystal form I |
| Methanol-ethyl acetate | 1:1 | Crystal form I |
| Methanol-ethyl acetate | 1:4 | Crystal form I |
| Methanol-ethyl acetate | 4:1 | Crystal form I |
| Methanol-isopropyl acetate | 1:1 | Mixed crystal forms |
| Methanol-isopropyl acetate | 1:4 | Mixed crystal forms |
| Methanol-isopropyl acetate | 4:1 | Mixed crystal forms |
| Methanol-methyl tert-butyl ether | 1:1 | Crystal form I |
| Methanol-methyl tert-butyl ether | 1:4 | Crystal form I |
| Methanol-methyl tert-butyl ether | 4:1 | Crystal form I |
| Methanol-tetrahydrofuran | 1:1 | Crystal form I |
| Methanol-tetrahydrofuran | 1:4 | Amorphous |
| Methanol-tetrahydrofuran | 4:1 | Crystal form I |
| Methanol-N-methylpyrrolidone | 1:1 | NS |
| Methanol-N-methylpyrrolidone | 1:4 | NS |
| Methanol-N-methylpyrrolidone | 4:1 | Crystal form I |
| Water-isopropanol | 1:1 | Crystal form I |
| Water-isopropanol | 1:4 | Crystal form I |
| Water-isopropanol | 4:1 | Crystal form I |
| Water-acetonitrile | 1:1 | Crystal form I |
| Water-acetonitrile | 1:4 | Crystal form I |
| Water-acetonitrile | 4:1 | Crystal form I |
| Water-acetone | 1:1 | Crystal form I |
| Water-acetone | 1:4 | Crystal form I |
| Water-acetone | 4:1 | Crystal form I |
| Water-methyl tert-butyl ether | 1:1 | Crystal form I |
| Water-methyl tert-butyl ether | 1:4 | Crystal form I |
| Water-methyl tert-butyl ether | 4:1 | Crystal form I |
| Water-tetrahydrofuran | 1:1 | Crystal form I |
| Water-tetrahydrofuran | 1:4 | Crystal form I |
| Water-tetrahydrofuran | 4:1 | Crystal form I |
| Water-N-methylpyrrolidone | 1:1 | Crystal form I |
| Water-N-methylpyrrolidone | 1:4 | Amorphous |
| Water-N-methylpyrrolidone | 4:1 | NS |

| | | |
|---|---|---|
| NS indicates that no solid was obtained during the experiment. | | |

### Example 5 Grinding Crystallization Method

50 mg of the compound represented by formula I was weighed and placed in a mortar. A small amount of solvent was added dropwise, and the mixture was ground. The PXRD patterns after grinding with different solvents for 30 minutes were tested respectively to investigate whether different crystal forms were produced. The results are shown in Table 6:

**Table 6 Experimental Results of Grinding Crystallization Method**

| **Solvent** | **Result** |
|---|---|
| Isopropanol | Crystal form I |
| Acetonitrile | Crystal form I |
| Acetone | Crystal form I |
| Ethyl acetate | Crystal form I |
| Isopropyl acetate | Crystal form I |
| Methyl tert-butyl ether | Crystal form I |
| Tetrahydrofuran | Crystal form I |
| N-methylpyrrolidone | Crystal form I |
| n-hexane | Crystal form I |
| Cyclohexane | Crystal form I |

### Example 6 Rapid Cooling Crystallization Method

50 mg of the compound represented by formula I was weighed and placed in a beaker, and an appropriate amount of solvent was added to dissolve the compound completely. The resulting mother liquor was placed in a 40°C water bath for 20 minutes, and was then taken out and immediately placed in an ice-water mixture (4°C). If a solid precipitated, the supernatant was removed, and the solid was dried and analyzed by PXRD. The results are shown in Table 7:

**Table 7 Experimental Results of Rapid Cooling Crystallization Method**

| **Solvent** | **Result** |
|---|---|
| Methanol | Crystal form I |
| Ethanol | Amorphous |
| Acetic acid | Amorphous |
| Dimethyl sulfoxide | Amorphous |
| N, N-dimethylformamide | NS |
| Water | NS |
| Formic acid | Amorphous |

| | |
|---|---|
| NS indicates that no solid was obtained during the experiment. | |

### Example 7 Vapor Diffusion Method

50 mg of the compound represented by formula I was weighed into a sample vial, and an appropriate amount of solvent was added to form a clear solution. The sample vial was placed open inside another larger glass container (outer container). A poor solvent was added to the outer container. The outer container was capped and tightened (or sealed with a sealing film), and was allowed to stand and grow at room temperature, allowing the outer solvent to continuously vaporize and diffuse into the inner container, thereby reducing the solubility of the sample in the mixed solvent system and causing crystals to precipitate. Whether different crystal forms were produced was investigated. The results are shown in Table 8:

**Table 8 Experimental Results of Vapor Diffusion Method**

| **Solvent** | **Result** |
|---|---|
| Isopropanol-methanol | Crystal form I |
| Isopropanol-acetic acid | NS |
| Isopropanol-water | Crystal form I |
| Isopropanol-formic acid | NS |
| Acetonitrile-methanol | Crystal form I |
| Acetonitrile-acetic acid | NS |
| Acetonitrile-water | Crystal form I |
| Acetonitrile-formic acid | NS |
| Acetone-methanol | Crystal form I |
| Acetone-acetic acid | Crystal form I |
| Acetone-water | Crystal form I |
| Acetone-formic acid | NS |
| Ethyl acetate-methanol | Crystal form I |
| Ethyl acetate-acetic acid | NS |
| Ethyl acetate-water | Crystal form I |
| Ethyl acetate-formic acid | NS |
| Isopropyl acetate-methanol | Crystal form I |
| Isopropyl acetate-acetic acid | NS |
| Isopropyl acetate-water | Crystal form I |
| Isopropyl acetate-formic acid | NS |
| Methyl tert-butyl ether-methanol | Crystal form I |
| Methyl tert-butyl ether-acetic acid | NS |
| Methyl tert-butyl ether-water | Crystal form I |
| Methyl tert-butyl ether-formic acid | NS |
| Tetrahydrofuran-methanol | Crystal form I |
| Tetrahydrofuran-acetic acid | NS |

| | |
|---|---|
| NS indicates that no solid was obtained during the experiment. | |

### Example 8 Single Crystal Growth

In the binary mixed-solvent crystallization experiment, under a solvent condition of water-acetone (1:4, v/v), colorless, transparent, strip-like crystals that met the requirements for a single-crystal X-ray diffraction experiment were obtained after slow evaporation for about 20 days. Measurement was carried out according to the first method of Volume IV, General Chapter 0451 in the Chinese Pharmacopoeia, 2020 edition, with test conditions of: CuKα radiation, and φ/ω scanning. In the single crystal diffraction experiment, a total of 18678 reflections were collected, with 10233 independent reflections, and 9936 observable reflections (|F|2 ≥ 2σ|F|2). The crystal structure was solved by the direct method (Shelxs97), and the results are shown in Table 9:

**Table 9 Unit Cell Parameters of Crystal Form I Single Crystal**

| **Parameter** | | **Value** |
|---|---|---|
| Crystal system | | Triclinic |
| Space group | | P1 |
| Unit cell parameters | a(Å) | 9.1081(1) |
| | b(Å) | 14.8986(2) |
| | c(Å) | 17.5130(2) |
| | α(°) | 101.807(1) |
| | β(°) | 93.001(1) |
| | γ(°) | 90.441(1) |
| Unit cell volume V(Å³) | | 2322.59(5) |
| Z (number of asymmetric units in the unit cell) | | 1 |
| Calculated density (g/cm³) | | 1.214 |

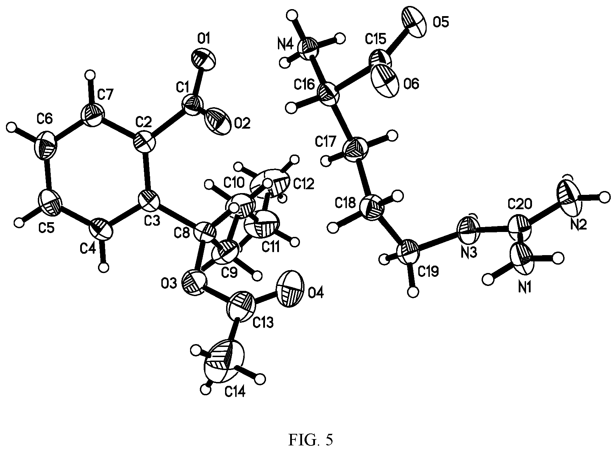

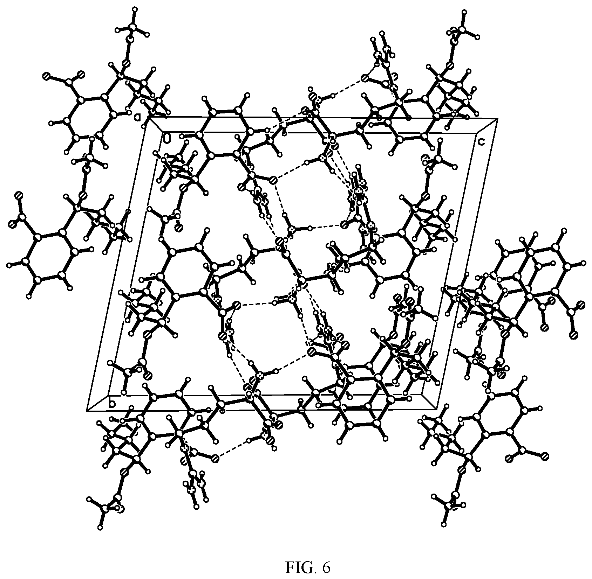

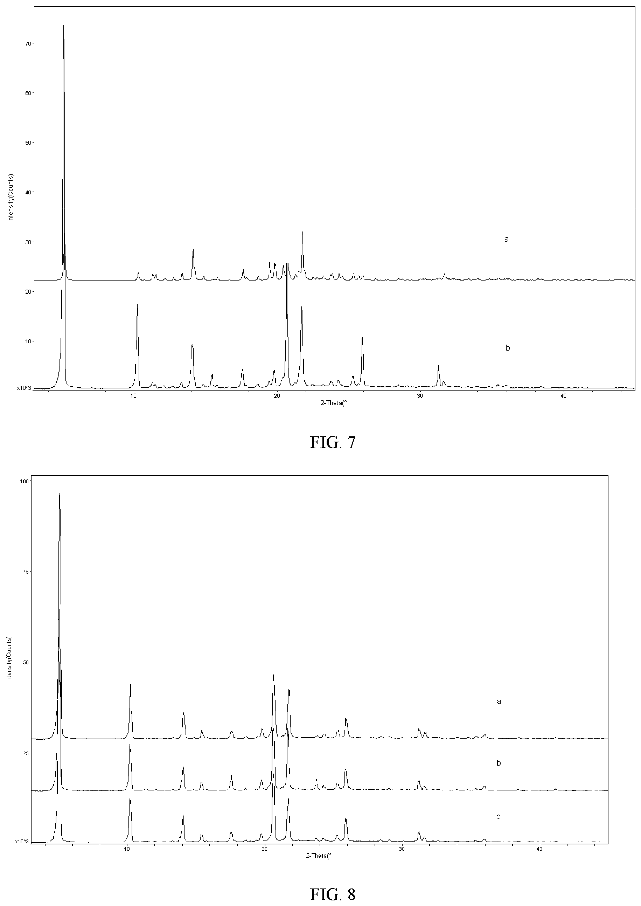

The results showed that: the molecular arrangement in the crystal state belongs to the space group P1; the sample should be optically active; the Flack coefficient is 0.06(13), and the absolute configuration of the compound in the crystal can be determined. In the crystal state, the molecules are maintained in a stable spatial arrangement through hydrogen bonds and van der Waals forces. An ellipsoid plot of the stereochemical structure of an asymmetric unit is shown in FIG. 4, an ellipsoid plot of the stereochemical structure of a single molecule is shown in FIG. 5, and a projection diagram of the unit cell packing viewed along the a-axis is shown in FIG. 6. Using the above single crystal structure data, a corresponding simulated powder diffraction pattern was calculated and compared with the pattern of crystal form I. The results showed that the two crystal forms are consistent (see FIG. 7 for details). In FIG. 7, curve a is the simulated powder pattern from the single crystal of the compound, and curve b is the PXRD pattern of crystal form I.

### Comparative Example 1

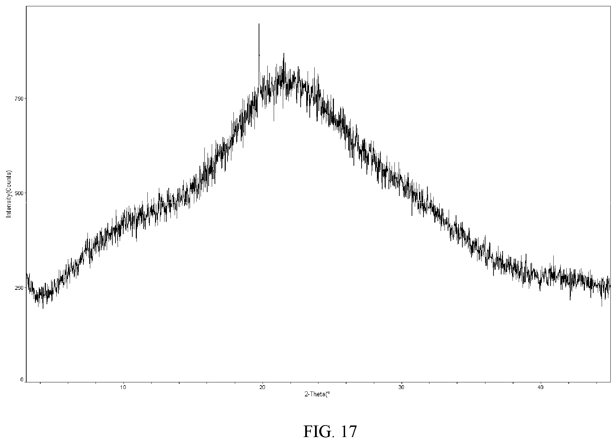

The compound represented by formula I was prepared according to the method disclosed in Example 10 of WO2020107500A1. The physical state was a solid, and the solvent f was 95% ethanol. After recrystallization, PXRD results showed that the solid was amorphous (see FIG. 17 for details).

### Example 9 Crystal Form Stability Study

Samples of crystal form I were subjected to physical stability tests under different storage conditions. The storage conditions were respectively:
(1) High temperature: 60°C
(2) High humidity: RH 90% ± 5%
(3) Under light exposure: 4500 lx ± 500 lx, with the total illumination from the light source reaching 1.2×10⁶ lux•hr

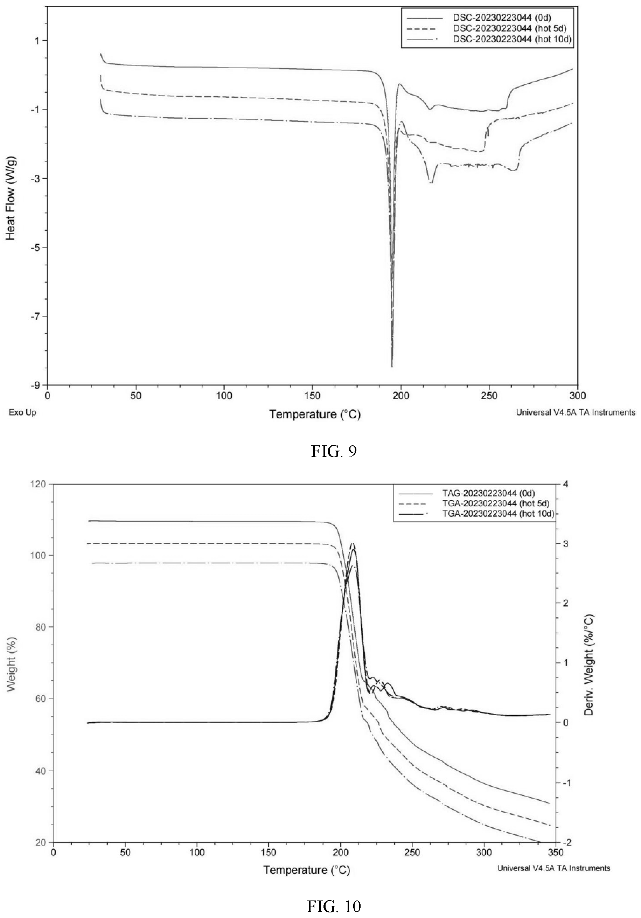

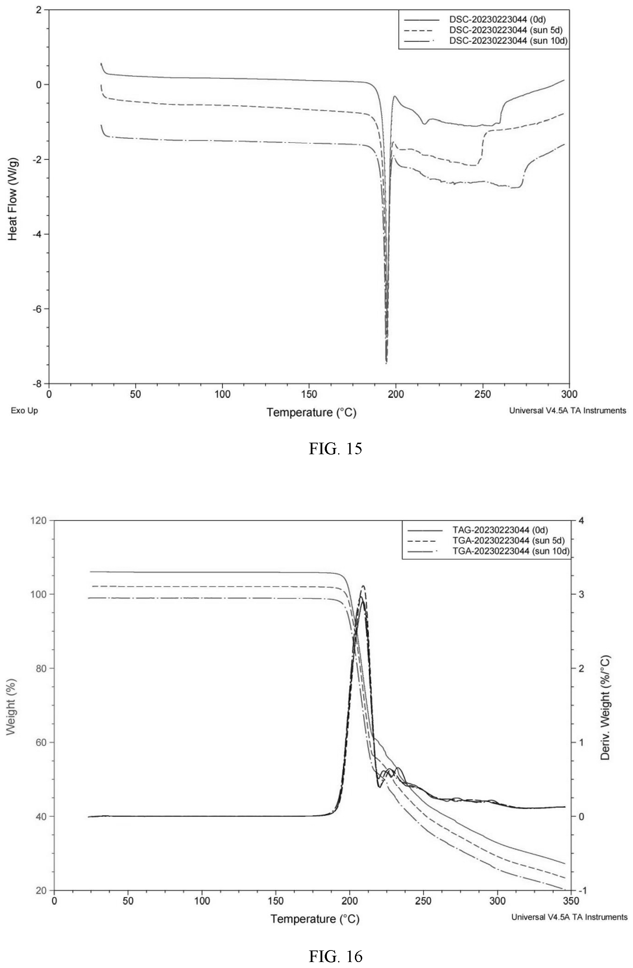

PXRD, DSC, and TGA tests were performed on the samples on day 0, day 5, and day 10, respectively, to investigate the stability of the samples under high temperature, high humidity, and light exposure conditions. The experimental results are detailed in FIG. 8 to FIG. 16 of the description.

In FIG. 8, curve a is for day 0, curve b is for day 5, and curve c is for day 10.

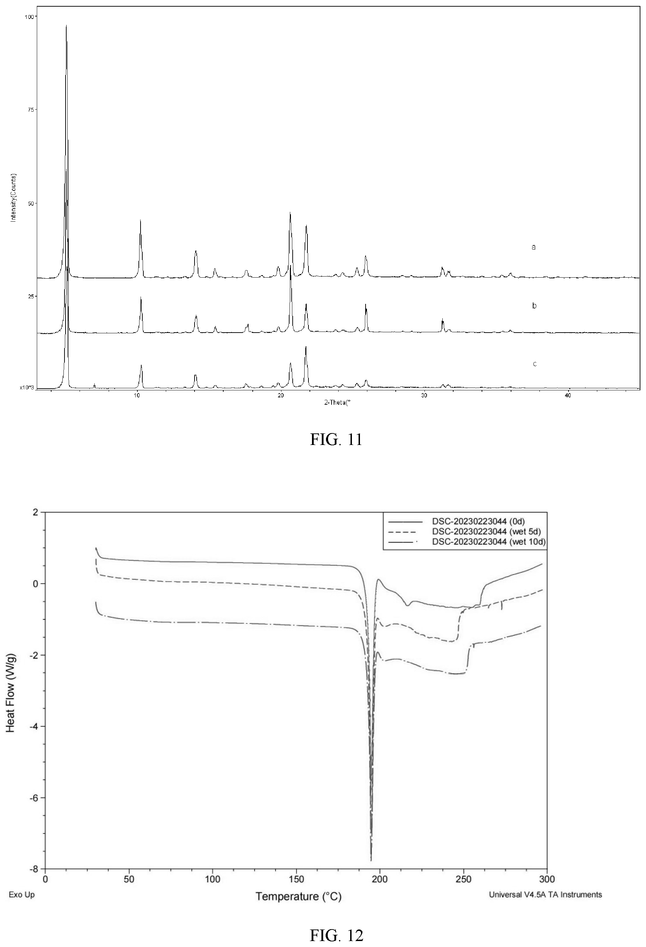

In FIG. 11, curve a is for day 0, curve b is for day 5, and curve c is for day 10.

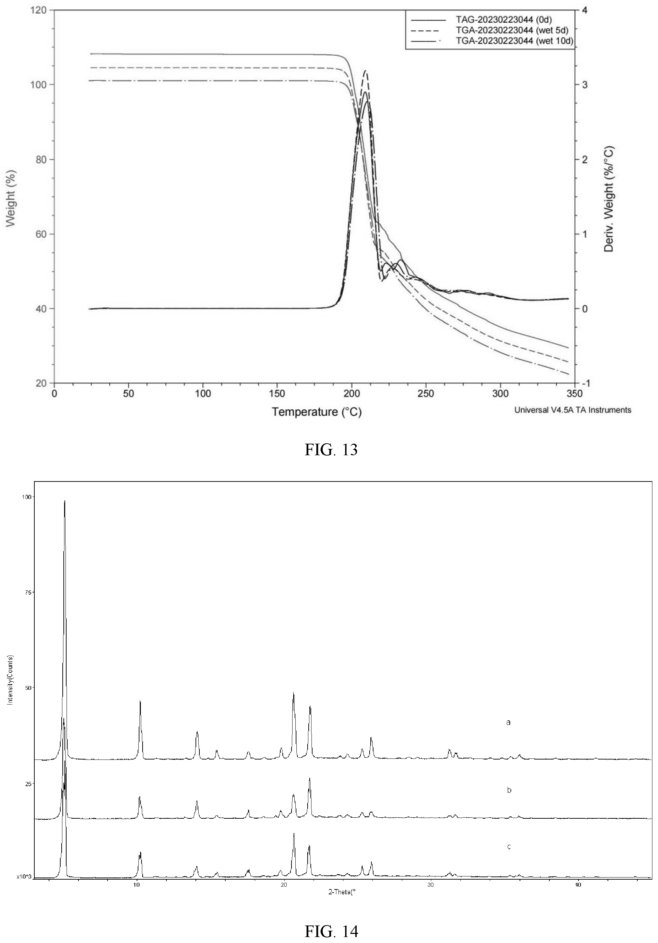

In FIG. 14, curve a is for day 0, curve b is for day 5, and curve c is for day 10.

Conclusion: Under conditions of high temperature (60°C), high humidity (RH 90% ± 5%), and light exposure (4500 lx ± 500 lx, with the total illumination reaching 1.2×10⁶ lux•hr), the powder diffraction pattern, DSC trace, and TGA trace of crystal form I showed no significant difference, indicating that its stability was good and no crystal form transformation occurred.

### Example 10

Samples of crystal form I (25 mg each) were weighed, placed into intrinsic dissolution rate metal modules, and maintained at a pressure of 20 pounds for 30s and then placed in 20 mL of SGF (Simulated Gastric Fluid) or FeSSIF (Fed-State Simulated Intestinal Fluid) physiological medium, respectively. The medium was maintained at 37°C and a rotation speed at 100 rpm throughout the process. Then, at 5, 15, 30, 45, 60, 90, and 120 min, respectively, a 0.5 mL aliquot of the solution was taken and filtered through a 0.22 µm aqueous microfiltration membrane. And the volume was replenished with 0.5 mL of the corresponding physiological medium. The concentration was determined according to an HPLC content assay method.

The results showed that: the dissolution rate of crystal form I was significantly higher than that of the solid obtained in Comparative Example I.

The descriptions of the above examples are only for the purpose of helping to understand the method and core ideas of the present disclosure. It should be noted that, for a person of ordinary skill in the art, several improvements and modifications can be made to the present disclosure without departing from the principles of the present disclosure. These improvements and modifications also fall within the scope of protection of the claims of the present disclosure.

## Claims

1. A crystal form I of L-arginine *(S)*-2-(1-acetoxy-n-pentyl)benzoate as represented by formula I, wherein an X-ray powder diffraction pattern of the crystal form I, measured using Cu-Kα radiation, has characteristic peaks at diffraction angles 2θ of 5.10, 10.23, 14.13, 17.58, 19.81, 20.63, and 21.73, with an error range of ±0.2 for each characteristic peak 2θ,

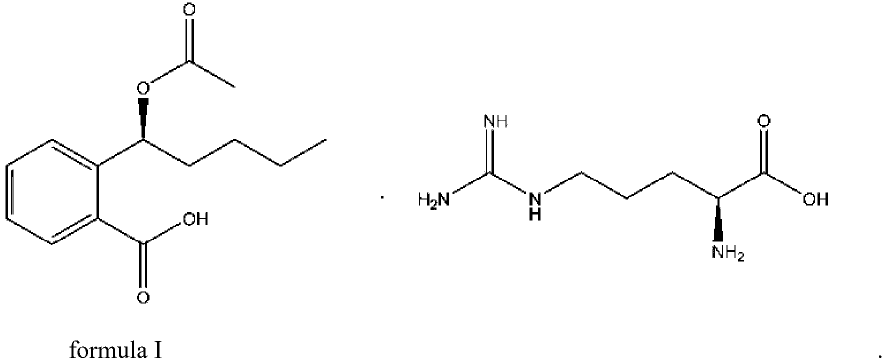

2. The crystal form I of L-arginine *(S)*-2-(1-acetoxy-n-pentyl)benzoate as represented by formula I according to claim 1, wherein the X-ray powder diffraction pattern has characteristic peaks at diffraction angles 2θ of 5.10, 10.23, 14.13, 15.42, 17.58, 19.81, 20.63, 21.73, and 25.88, with an error range of ±0.2 for each characteristic peak 2θ.

3. The crystal form I of L-arginine *(S)*-2-(1-acetoxy-n-pentyl)benzoate as represented by formula I according to claim 1, wherein the X-ray powder diffraction pattern has characteristic peaks at diffraction angles 2θ of 5.10, 10.23, 14.13, 15.42, 17.58, 19.81, 20.63, 21.73, 24.28, 25.36, 25.88, 31.21, and 31.65, with an error range of ±0.2 for each characteristic peak 2θ.

4. The crystal form I of L-arginine *(S)*-2-(1-acetoxy-n-pentyl)benzoate as represented by formula I according to claim 1, wherein the X-ray powder diffraction pattern has characteristic peaks at diffraction angles 2θ of 5.10, 10.23, 11.30, 14.13, 15.42, 17.58, 19.81, 20.63, 21.73, 24.28, 25.36, 25.88, 31.21, and 31.65, with an error range of ±0.2 for each characteristic peak 2θ.

5. The crystal form I of L-arginine *(S)*-2-(1-acetoxy-n-pentyl)benzoate represented by formula I according to claim 1, wherein the crystal form I of L-arginine *(S)*-2-(1-acetoxy-n-pentyl)benzoate represented by formula I has a melting point of 192°C; and the crystal form I of L-arginine *(S)*-2-(1-acetoxy-n-pentyl)benzoate represented by formula I has a decomposition temperature of 195°C.

6. A method for producing the crystal form I of L-arginine *(S)*-2-(1-acetoxy-n-pentyl)benzoate represented by formula I according to any one of claims 1 to 5, wherein the method is selected from any one of Method 1 to Method 7 below:
Method 1: a room temperature evaporation method, comprising dissolving the compound of formula I in a good solvent, allowing the solvent to evaporate from an open container at room temperature, crystallizing, filtering, and drying to obtain the crystal form I of the compound of formula I;
Method 2: a suspending crystallization method, comprising mixing the compound of formula I with a poor solvent to form a suspension, shaking the suspension at room temperature or at 40°C to 60°C, removing the solvent, and drying to obtain the crystal form I of the compound of formula I;
Method 3: a dissolution-precipitation crystallization method, comprising dissolving the compound of formula I in a good solvent, adding a poor solvent, crystallizing, filtering, and drying to obtain the crystal form I of the compound of formula I;
Method 4: a binary mixed-solvent crystallization method, comprising dissolving the compound of formula I in a binary mixed solvent, crystallizing, filtering, and drying to obtain the crystal form I of the compound of formula I, wherein the binary mixed solvent is a mixed solvent of a good solvent and a poor solvent;
Method 5: a grinding crystallization method, comprising mixing and grinding the compound of formula I with a poor solvent to obtain the crystal form I of the compound of formula I;
Method 6: a rapid cooling crystallization method, comprising dissolving the compound of formula I in a good solvent, crystallizing at a low temperature, filtering, and drying to obtain the crystal form I of the compound of formula I; and
Method 7: a vapor diffusion method, comprising dissolving the compound of formula I in a good solvent, placing the solution in an open container in an atmosphere of a poor solvent, allowing the solution to stand at room temperature, crystallizing, filtering, and drying to obtain the crystal form I of the compound of formula I.

7. The method according to claim 6, wherein,
in Method 1, the good solvent is a single solvent or a mixed solvent of one or more selected from the group consisting of water, a first alcohol solvent, a sulfoxide solvent, an amide solvent, and a carboxylic acid solvent; wherein the first alcohol solvent is selected from the group consisting of methanol and ethanol; the sulfoxide solvent is selected from dimethyl sulfoxide; the amide solvent is selected from the group consisting of N,N-dimethylformamide and N,N-dimethylacetamide; the carboxylic acid solvent is selected from the group consisting of formic acid, acetic acid, and benzoic acid; and the crystallizing is performed by a method selected from the group consisting of room temperature crystallization, cooling crystallization, solvent evaporation crystallization, and seed-induced crystallization;
in Method 2, the poor solvent is a single solvent or a mixed solvent of one or more selected from the group consisting of an ester solvent, an ether solvent, an aliphatic hydrocarbon solvent, an alicyclic hydrocarbon solvent, a second alcohol solvent, a nitrile solvent, and a ketone solvent; wherein the ester solvent is selected from the group consisting of ethyl acetate, isopropyl acetate, and butyl acetate; the ether solvent is selected from the group consisting of tetrahydrofuran, 1,4-dioxane, propylene glycol methyl ether, and methyl tert-butyl ether; the aliphatic hydrocarbon solvent is selected from the group consisting of n-hexane and n-heptane; the alicyclic hydrocarbon solvent is selected from cyclohexane; the second alcohol solvent is selected from isopropanol; the nitrile solvent is selected from acetonitrile; and the ketone solvent is selected from the group consisting of acetone and N-methylpyrrolidone;
in Method 3, the good solvent is selected from the group consisting of water, a first alcohol solvent, a carboxylic acid solvent, a sulfoxide solvent, and an amide solvent; wherein the first alcohol solvent is selected from the group consisting of methanol and ethanol; the carboxylic acid solvent is selected from the group consisting of formic acid and acetic acid; the sulfoxide solvent is selected from dimethyl sulfoxide; and the amide solvent is selected from the group consisting of N,N-dimethylformamide and N,N-dimethylacetamide; the poor solvent is selected from the group consisting of a second alcohol solvent, an ether solvent, an ester solvent, a ketone solvent, and a nitrile solvent; wherein the second alcohol solvent is selected from isopropanol; the ether solvent is selected from the group consisting of tetrahydrofuran, 1,4-dioxane, propylene glycol methyl ether, and methyl tert-butyl ether; the ester solvent is selected from the group consisting of ethyl acetate, isopropyl acetate, and butyl acetate; the ketone solvent is selected from the group consisting of acetone and N-methylpyrrolidone; the nitrile solvent is selected from acetonitrile; and the crystallizing is performed by a method selected from the group consisting of room temperature crystallization, cooling crystallization, solvent evaporation crystallization, and seed-induced crystallization;
in Method 4, the good solvent is selected from the group consisting of a first alcohol solvent and water; wherein the first alcohol solvent is selected from the group consisting of methanol and ethanol; the poor solvent is selected from the group consisting of a second alcohol solvent, a nitrile solvent, a ketone solvent, an ester solvent, and an ether solvent; wherein the second alcohol solvent is selected from isopropanol; the nitrile solvent is selected from acetonitrile; the ketone solvent is selected from the group consisting of acetone and N-methylpyrrolidone; the ester solvent is selected from the group consisting of ethyl acetate, isopropyl acetate, and butyl acetate; and the ether solvent is selected from the group consisting of tetrahydrofuran, 1,4-dioxane, propylene glycol methyl ether, and methyl tert-butyl ether;
in Method 5, the poor solvent is selected from the group consisting of a second alcohol solvent, a nitrile solvent, a ketone solvent, an ester solvent, an ether solvent, a ketone solvent, an aliphatic hydrocarbon solvent, and an alicyclic hydrocarbon solvent; wherein the second alcohol solvent is selected from isopropanol; the nitrile solvent is selected from acetonitrile; the ketone solvent is selected from the group consisting of acetone and N-methylpyrrolidone; the ester solvent is selected from the group consisting of ethyl acetate, isopropyl acetate, and butyl acetate; the ether solvent is selected from the group consisting of tetrahydrofuran, 1,4-dioxane, propylene glycol methyl ether, and methyl tert-butyl ether; the aliphatic hydrocarbon solvent is selected from the group consisting of n-hexane and n-heptane; and the alicyclic hydrocarbon solvent is selected from cyclohexane;
in Method 6, the good solvent is selected from the group consisting of a first alcohol solvent, a carboxylic acid solvent, a sulfoxide solvent, an amide solvent, and water; wherein the first alcohol solvent is selected from the group consisting of methanol and ethanol; the sulfoxide solvent is selected from dimethyl sulfoxide; the amide solvent is selected from the group consisting of N,N-dimethylformamide and N,N-dimethylacetamide; and the carboxylic acid solvent is selected from the group consisting of formic acid, acetic acid, and benzoic acid; and
in Method 7, the good solvent is selected from the group consisting of water, a first alcohol solvent, and a carboxylic acid solvent; wherein the first alcohol solvent is selected from the group consisting of methanol and ethanol; and the carboxylic acid solvent is selected from the group consisting of formic acid and acetic acid; the poor solvent is selected from the group consisting of a second alcohol solvent, an ether solvent, an ester solvent, a ketone solvent, and a nitrile solvent; wherein the second alcohol solvent is selected from isopropanol; the ether solvent is selected from the group consisting of tetrahydrofuran, 1,4-dioxane, propylene glycol methyl ether, and methyl tert-butyl ether; the ester solvent is selected from the group consisting of ethyl acetate, isopropyl acetate, and butyl acetate; the ketone solvent is selected from the group consisting of acetone and N-methylpyrrolidone; and the nitrile solvent is selected from acetonitrile.

8. A pharmaceutical composition, comprising the crystal form I of L-arginine *(S)*-2-(1-acetoxy-n-pentyl)benzoate as represented by formula I according to any one of claims 1 to 5 and a pharmaceutically acceptable adjuvant.

9. Use of the crystal form I of L-arginine *(S)*-2-(1-acetoxy-n-pentyl)benzoate as represented by formula I according to any one of claims 1 to 5 or the pharmaceutical composition according to claim 8 in the manufacture of a medicament for treating an ischemic cardiovascular or cerebrovascular disease.

10. The use according to claim 9, wherein the ischemic cardiovascular or cerebrovascular disease is selected from stroke; preferably cerebral stroke; more preferably ischemic cerebral stroke.
